# EUROPEAN PATENT APPLICATION

(11) **EP 2 063 273 A1**
(43) Date of publication of application: **27.05.2009**
(21) Application number: 07121222.9
(22) Date of filing: 21.11.2007
(51) Int. Cl.: G01N 33/86

(54) **Method for assessing the fibrinogen contribution in coagulation**

(71) Applicant: Pentapharm GmbH, 81829 München (DE)
(72) Inventor: Schubert, Axel, 81377 München (DE); Schwaiger, Martin, 81667 München (DE); Kessler, Max, 81543 München (DE)
(74) Representative: Peckmann, Ralf

(57) **Abstract**

The present invention is directed to a diagnostic method for the determination of a coagulopathy in a patient, in particular, for calculating the individual need of blood components, preferably blood platelets and/or fibrinogen, which has to be substituted in a patient.

## Description

### FIELD OF THE INVENTION

The present invention is directed to a diagnostic method for the determination of a coagulopathy in a patient, in particular, for determining the individual need of blood components, preferably fibrinogen, which should be substituted in a patient to balance haemostasis.

### BACKGROUND

It is essential for survival that a wound stops bleeding, i.e. that the body possesses an adequate mechanism for haemostasis. The process of blood clotting can be activated in the case of injuries or inflammations by either extrinsic or intrinsic factors, e.g. tissue factor (TF) or Hagemann factor (F XII), respectively. Both activation channels are continued in a common branch of the cascade resulting in thrombin formation. The thrombin itself finally initiates the formation of fibrin fibres which represent the protein backbone of blood clots.

The other main constituent of the final blood clot are the thrombocytes which are interconnected by the fibrin fibres and undergo a number of physiological changes during the process of coagulation. Within limits a lack of thrombocytes can be substituted by an increased amount of fibrin or vice versa. This is reflected in the observation that the thrombocyte counts as well as the fibrinogen concentration varies even within a healthy population.

Various methods have been introduced to assess the potential of blood to form an adequate clot and to determine the blood clots stability. Common laboratory tests such as thrombocyte counts or the determination of fibrin concentration provide information on whether the tested component is available in sufficient amount but lack in answering the question whether the tested component works properly under physiological conditions (e.g. the polymerisation activity of fibrinogen under physiological conditions can not be assessed by common optical methods). Besides that, most laboratory tests work on blood-plasma and therefore require an additional step for preparation and additional time which is unfavourable especially under POC (point of care) conditions.

Another group of tests which overcomes these problems is summarized by the term "viscoelastic methods". The common feature of these methods is that the blood clot firmness (or other parameters dependent thereon) is continuously determined, from the formation of the first fibrin fibres until the dissolution of the blood clot by fibrinolysis. Blood clot firmness is a functional parameter, which is important for haemostasis in vivo, as a clot must resist blood pressure and shear stress at the site of vascular injury. Clot firmness results from multiple interlinked processes: coagulation activation, thrombin formation, fibrin formation and polymerization, platelet activation and fibrin-platelet interaction and can be compromised by fibrinolysis. Thus, by the use of viscoelastic monitoring all these mechanisms of the coagulation system can be assessed.

A common feature of all these methods used for coagulation diagnosis is that the blood clot is placed in the space between a cylindrical pin and an axially symmetric cup and the ability of the blood clot to couple those two bodies is determined.

The first viscoelastometric method was called "thrombelastography" (Hartert H: Blutgerinnungsstudien mit der Thrombelastographie, einem neuen Untersuchungsverfahren. Klin Wochenschrift 26:577-583, 1948). In the thromboelastography, the sample is placed in a cup that is periodically rotated to the left and to the right by about 5°, respectively. A pin is freely suspended by a torsion wire. When a clot is formed it starts to transfer the movement of the cup to the pin against the reverse momentum of the torsion wire. The movement of the pin as a measure for the clot firmness is continuously recorded and plotted against time. For historical reasons the firmness is measured in millimetres.

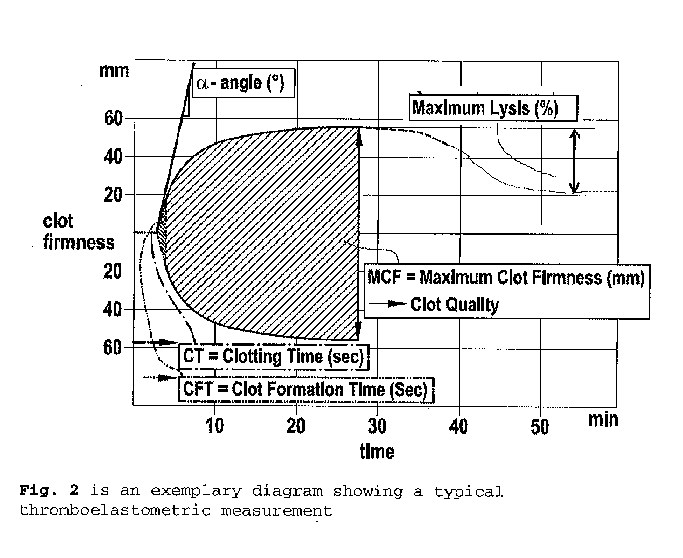

The result of a typical measurement of this kind is illustrated in Figure 2. One of the most important parameters is the time between the activator induced start of the coagulation cascade and the time until the first long fibrin fibres have been build up which is indicated by the firmness signal exceeding a defined value. This parameter will be called clotting time or just CT in the following. Another important parameter is the clot formation time (CFT) which gives a measure for the velocity of the development of a clot. The CFT is defined as the time it takes for the clot firmness to increase from 2 to 20 mm. The maximum firmness a clot reaches during a measurement, further on referred to as maximum clot firmness or just MCF, is also of great diagnostic importance.

Modifications of the original thromboelastography technique (Hartert et al. (US 3,714,815) have been described by Cavallari et al. (US 4,193,293), by Do et al. (US 4,148,216), by Cohen (US 6,537.819), further modifications by Calatzis et al. (US 5,777,215) are called thromboelastometry.

During coagulation the fibrin backbone creates a mechanical elastic linkage between the surfaces of the blood-containing cup and a pin plunged therein. A proceeding coagulation process induced by adding one or more activating factor(s) can thus be observed. In this way, various deficiencies of a patient's haemostatic status can be revealed and can be interpreted for proper medical intervention.

A general advantage of viscoelastometric, e.g. thromboelastometric, techniques compared to other laboratory methods in this field therefore is that the coagulation process and the change of mechanical properties of the sample are monitored as a whole. This means that - in contrary to other laboratory methods mentioned above - viscoelastometry does not only indicate if all components of the coagulation pathways are available in sufficient amounts but also if each component works properly.

The possibility to activate or to inhibit certain components of the coagulation system is especially useful in conjunction with state-of-the-art thromboelastometers such as the ROTEM (Pentapharm GmbH, Munich, Germany) which allows conducting four measurements in parallel. This allows to achieve detailed information on the current status of the coagulation-situation of a patient and therefore allows an appropriate therapy within several minutes.

This is of particular importance in case of patients struck by massive blood loss as it often occurs in context with multiple traumata or major surgery. The blood of such patients often is diluted due to infusions which are administered to replace the loss in volume. This leads to a decrease of the concentration of thrombocytes as well as coagulation factors including fibrinogen.

Fibrinogen is activated by thrombin and polymerizes to fibrin which contributes to the final firmness of the developing blood clot. In normal blood the fibrin network contributes about one third to the overall firmness of the blood clot.

A coagulopathy can be caused for example by an excess or a deficiency of thrombocytes, fibrinogen, activating factors or other blood components or a dysfunction of these elements.

Hence, it is important to determine at which point of the coagulation pathway a problem occurs to choose an appropriate medication.

A topic of outstanding importance in this context is the determination of the fibrin networks contribution to the final stability of a growing blood clot. This can be achieved by adding a thrombocyte inhibitor, e.g. cytochalasin D or abciximab, to the sample before measurement. That way the partial contribution of fibrin to the total clot firmness becomes directly accessible.

If the result of such a test is within the range of corresponding tests of healthy people but a comparative viscoelastometric measurement conducted without inhibiting the thrombocyte function shows decreased clot firmness and the patient is bleeding, donation of thrombocyte concentrates will be the appropriate therapy. If, on the other hand, the inhibited sample exhibits a decreased firmness in comparison to thrombocyte inhibited samples of normal population, the administration of thrombocytes will not stop bleeding, but the administration of purified fibrinogen or fibrinogen contained in other blood substitution products like freshly frozen plasma (FFP) seems appropriate.

Thromboelastometric measurements may become less sensitive if the total firmness of the sample is low. This situation especially occurs if the thrombocytes in the blood sample are inactivated to assess the partial fibrin contribution to clot firmness (such as mentioned above by administration of, e.g., cytochalasin D or abciximab), since such tests naturally exhibit a lower total firmness. The situation becomes even worse if the patient blood is diluted due to the earlier administration of volume substitutes like NaCl or HES solutions.

The reason for a lower precision when testing the fibrinogen function of pathologic samples originates from applying the thromboelastometric method beyond the range of its initially intended application: The geometry of the standardized measurement cell (the outer diameter of the pin is about 6.0 mm and the space between cup and pin is about 1.0 mm) was originally designed to compare conventionally activated blood samples of normal and pathologic patients without additional thrombocyte inhibition. Such tests result in values for the maximum clot firmness (MCF) between 25 and 75 mm, which is a highly sensitive range for the method. In thrombocyte inhibited tests, however, only the fibrinogen contribution to the clot is measured, since the platelet functionality is completely suppressed. Hence, these tests yield lower MCF's (between about 10 and 25 mm for normal patients and well below 10 mm in the case of pathologic samples). Considering a minimum sensitivity level of about 1 mm for the current disposable geometry, higher test-to-test result variations (coefficient of variations) are a consequence when measuring such samples.

The magnitude of the measured signal is proportional to the torque being transmitted to the shaft of the instrument by elastic fibrin fibres between cup and pin walls. Therefore it depends on the blood volume as well as on the total area of the clot surface.

As a conventional solution, the measurement signal could be increased by increasing the sample amount if the expected clot firmness is rather low. However, this approach is limited to the amount of blood usually available for coagulation analysis in clinical practice. A further practical limitation of this approach is that the sample volume in the cup is limited due to the geometric dimensions of commercially available thromboelastometers. Furthermore, there are situations where the amount of blood available for analysis is further limited, especially in surgery on infants (due to ethical considerations) or in pharmaceutical industry where mice are used as donors. Beyond that, thromboelastometry in the pharmaceutical industry for drug development has an increased demand for high accuracy measurements on small samples.

Since a therapeutic decision about substitution of blood components bears several risks for the patient as well, the amount of substitute to be administered plays an important role with respect to therapy success and patient recovery. Accordingly, estimation of the proper amount of a therapeutic substitute, e.g. FFP or other compounds containing fibrinogen, is of major importance in the context of optimal patient treatment to balance haemostasis. The invention of a method to assess the required amount of a blood substitute on the basis of in-vitro diagnostics, however, requires also sufficient sensitivity of the underlying in-vitro measurement. For that reason, the optimization of the measurement sensitivity itself is directly connected to such a method and cannot be seen independently.

### SUMMARY OF THE INVENTION

It is a problem underlying the presented invention to provide a diagnostic method, which ends up in the determination of the amount of blood components which have to be substituted to a patient in need thereof, i.e. to determine the optimum therapeutic quantity of a coagulation component to properly balance impaired haemostasis. It is a further problem underlying the invention to provide a therapeutic method which comprises a determination of the individual coagulopathy of a patient, calculating the required amount of blood components, e.g. FFP or other blood products or compounds containing fibrinogen and/or other coagulation components, to be substituted to the patient and administering the required amount to said patient.

Directly connected to this invention is the problem to provide a method for viscoelastic measurements of thrombocyte inhibited blood samples with sufficiently high sensitivity and, therefore, accuracy of measurement compared to heretofore used viscoelastometric, i.e thromboelastometric or thromboelastographic, measurement systems.

These problems are solved by the subject-matter of the independent claims. Preferred embodiments are set forth in the dependent claims.

The present invention comprises an innovative solution to establish an algorithm calculating the required amount of above mentioned therapeutic substitutes to balance impaired haemostasis. This is to measure the contribution of polymerized fibrinogen to the total clot firmness in thrombocyte inhibited test samples. By preparation of samples with different, pre-determined fibrinogen content, certain diagnostic parameters as for example the clot firmness amplitude at a certain time after clotting initiation/clotting detection or also the maximum clot firmness (MCF), can be related to the fibrinogen deficiency/excess in these samples. An algorithm for calculating optimum amounts of substitute(s) from one or more suited diagnostic parameters can be established by taking into account experiments on blood samples of patients that have been treated with defined amounts of haemo-diluents. Haemo-diluents (e.g. colloids or other solutions) are usually administered to patients suffering from a significant loss of blood. This algorithm allows a proper treatment to rebalance haemostasis even if the origin of fibrinogen deficiency/dysfunction/inhibition is not known.

This algorithm can also include correction factors which are necessary to consider because the measurements have been performed under special conditions as described below.

Establishing a reliable method to assess the optimum therapeutic amount of a substitute to balance haemostasis requires a sufficient accuracy of viscoelastometric measurements. The present invention solves that problem by adding a certain (and preferably fixed) amount of a polymerizable substance to the blood sample. Said substance (e.g. purified fibrinogen) should polymerize in a similar way and timescale under the conditions of measurement. By this approach, the measurement signal of thrombocyte inhibited blood samples can be increased and shifted to a more sensitive range of any viscoelastometric measurement method. Of course, the contribution of the mentioned polymerizing substance to the overall firmness has to be considered when determining the amount of substitute to be administered on the basis of the viscoelastometric measurement.

By this means, the relative differences in firmness between pathological blood samples and samples of healthy people can be measured with increased accuracy. Optionally, the therapeutic decision might be simplified by a suitable algorithm in the instrument software adapted such that the (known) contribution of the added polymerizing substance is subtracted form the measured overall clot firmness. Thereby displaying the result to the operator in the same way as conventional thrombocyte inhibited tests while still benefiting from an increased accuracy. Additionally the deviation of the results of a thrombocyte inhibited tests from the corresponding results of healthy people might be used in order to calculate which amount of fibrinogen is necessary to balance haemostasis.

### BRIEF DESCRIPTION OF THE DRAWINGS

Further features and advantages of the present invention will be evident from a description of embodiments with reference to the figures.

### The figures are showing the following:

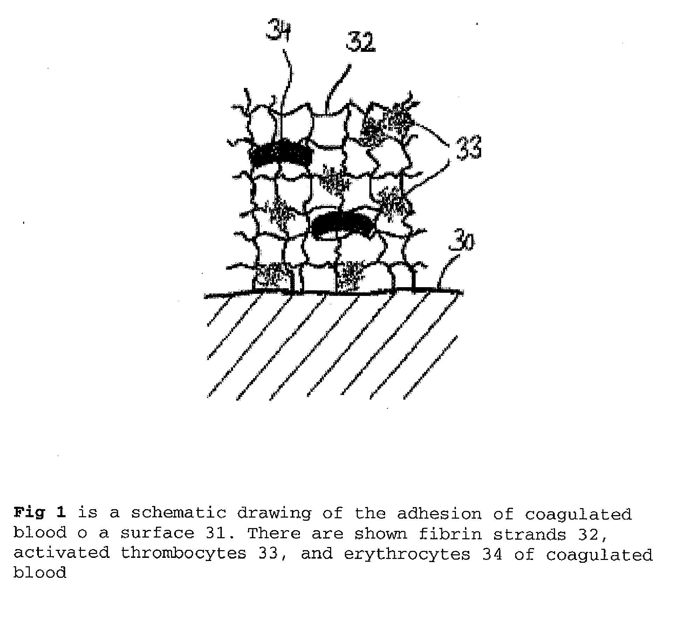

Fig. 1 is a schematic drawing of clot and fibrinogen network Fig. 2 is an exemplary diagram showing a typical thromboelastometric measurement.

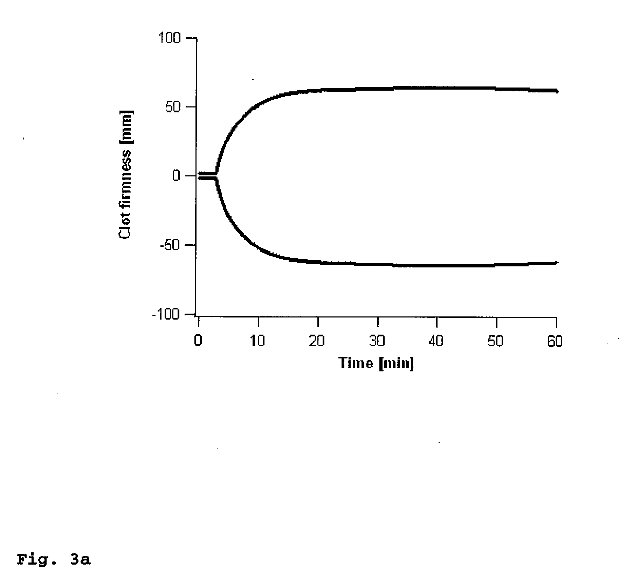

Fig. 3a is an exemplary diagram showing a typical thromboelastometric measurement of a healthy person without in-vitro thrombocyte inhibition.

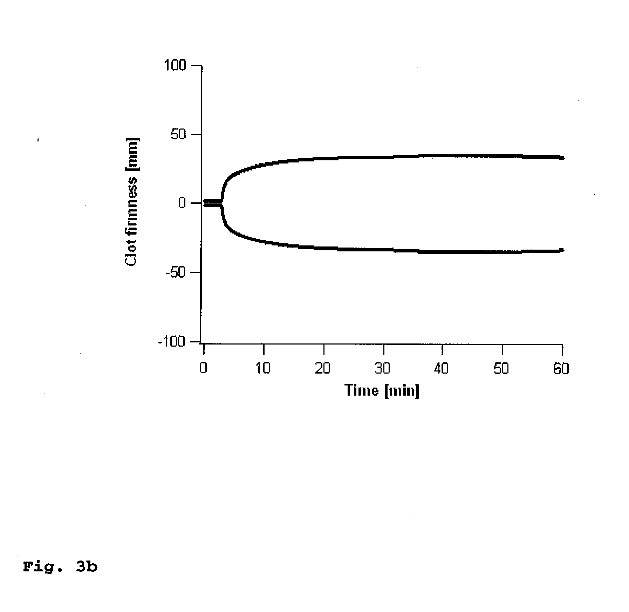

Fig. 3b is an exemplary diagram showing a typical thromboelastometric measurement of a pathologic patient without in-vitro thrombocyte inhibition.

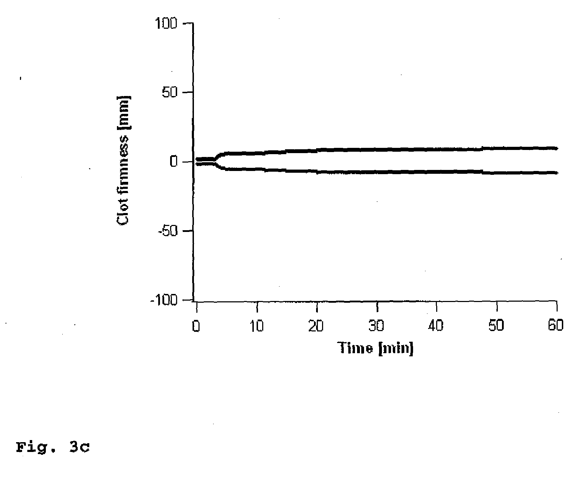

Fig. 3c is an exemplary diagram showing a typical thromboelastometric measurement of the same pathologic patient with in-vitro thrombocyte inhibition.

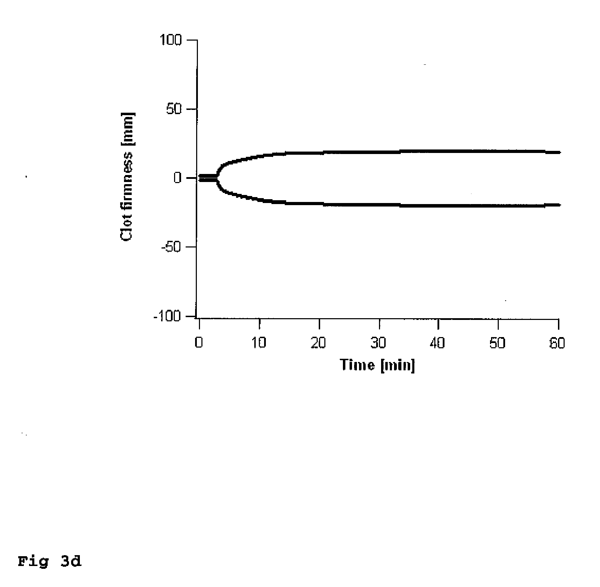

Fig. 3d is an exemplary diagram showing a typical thromboelastometric measurement of the same pathologic patient with in-vitro thrombocyte inhibition and an additional amount of polymerizable substance added in-vitro.

### DETAILED DESCRIPTION OF THE INVENTION

In a first aspect, the present invention provides a diagnostic method to determine a coagulopathy in a patient, comprising the steps of:
a) obtaining a blood sample from a patient;
b) adding a coagulation component inhibitor (e.g. platelet inhibitor) to the sample in a suitable amount for inhibiting the coagulation component function (e.g. platelet function) or adding the blood sample into a receptacle containing this amount of coagulation component inhibitor, respectively.
c) performing a viscoelastometric measurement, preferably by determining the clotting time, the clot formation time, the firmness of the clot over time, the maximum clot firmness and/or fibrinolysis from the blood sample under suitable conditions in a suitable device;
d) comparing the results obtained in step d) with reference data obtained from one or more other healthy and/or pathological blood donor(s);
   wherein differing results are indicative for the presence of a coagulopathy based on a deficiency in forming a proper blood clot and where the results can be used to estimate the amount substitute(s) required to balance haemostasis of said patient.

The present invention further intends to more specifically adapt viscoelastometric measurements performed under the condition of said coagulation component inhibition. Basically, this is performed before step c), wherein an agent is added which is able to polymerize and to thereby increase the blood clot firmness. As mentioned above, the firmness range of blood samples of normal patients which have been inhibited regarding thrombocytes is 9 to 25 mm. This is considerably lower than the firmness range of blood samples of normal patients without thrombocyte inhibition (50 - 72 mm). Pathological patient samples often have a firmness of less than 9 mm, thus lying below the most sensitive range of today's thromboelastometers. This method adaptation aims at ending up at an overall firmness of the developing blood clot in the range of between 20 and 80 mm, more preferably between 30 and 60 mm.

The agent that is able to polymerize and thereby to increase the blood clot firmness can be selected from any known and biocompatible polymer, which does not interfere or disturb the diagnostic method performed on the blood sample. In the most preferred cases this agent is fibrinogen.

There are differing ways to consider the additional amount of polymeric agent added to the sample when determining the required amount of therapeutic substitute to rebalance haemostasis of said patient from the obtained measurement parameters (e.g, clot formation time, the firmness of the clot over time, maximum clot firmness etc.). At first, it is possible to use a calculation algorithm to determine the results which would have been obtained in said sample without adding said polymeric agent. Or, as an alternative, the results can be directly compared to results obtained from blood samples of other healthy and/or pathologic donors, which have been obtained after adding the same predefined amount of the polymeric agent to these samples (resulting in a similar concentration).

As indicated, the viscoelastometric measurement is performed in a suitable device or apparatus. Such an apparatus preferably is a thromboelastometer or a thrombelastograph. As an example for a thromboelastometer the ROTEM-system may be used. Regarding the design of the ROTEM-system and how to use it, it is referred to the publication of US 5,777,215, incorporated herein by reference. Further devices are for example disclosed in US 6,537,819 or US 5,777,215, both incorporated also by reference.

In step d), the parameter results obtained for said patient sample in step c) are compared to those of other healthy and/or pathological donors. In principle, the results obtained from said other donors are obtained in the same way as the results for said patient. However, it is obvious that it is not necessary to perform these measurements in parallel to the measurement of the patient sample, but it is sufficient and more feasible to establish a series of measurements for blood samples obtained from other donors in advance and use it as reference values. Preferably, the results obtained from said other donors are derived from the same (or at least) comparable ethnical group as the patient and more preferably also other individual characteristics like patient age, existence of pregnancy etc. are taken into account. The background for this preferred embodiment is that the blood coagulation behaviour and characteristics may differ between different groups of patients and, therefore, might end in misleading result interpretation.

In a preferred embodiment, the coagulation component inhibitor used in step b) is a platelet inhibitor and more preferably is selected from the group consisting of a cyto-skeletton inhibitor or a GBIIb/IIIa antagonist and preferably is cytochalasin D, abciximab, or a combination thereof.

In a preferred embodiment, a further diagnostic measurement is performed, wherein steps c) and d) are applied to untreated blood samples of said patient (i.e., no coagulation component inhibitor and no polymerizable agent added to the blood sample). Combined interpretation of the results obtained with treated and untreated samples are indicative for the presence of a platelet based coagulopathy. The diagnostic method of the first aspect of the present invention is used to determine the contribution of the fibrin network to the overall firmness of the blood clot and to obtain the amount of fibrinogen required to balance haemostasis of said patient. The further measurement of this preferred embodiment may, in addition, determine whether the coagulation components to be substituted are thrombocytes. As mentioned above, the therapeutic decision about substitution of blood components bears several risks for the patients and therefore, not only the amount of a substitute to be administered but also its type plays an important role with respect to therapy success and patient recovery.

Results obtained in step d) may be used to determine the amount of blood components which have to be substituted to the patient. This can also be done by considering further parameters, for example the total blood volume or the body weight of said patient, respectively. Obviously, such parameters are also influencing the amount of blood components which have to be substituted to the patient in need thereof.

The diagnostic method of the present invention may be used for any mammal, for which the determination of a coagulopathy is desirable. Preferably, the diagnostic method is performed on a blood sample of a human patient. The blood sample can be whole blood or any fraction thereof, e.g. blood plasma extracted by centrifugation.

In a preferred embodiment, a coagulation activator is further added to the blood sample before or during step c). Such an activator of coagulation might be selected from intrinsic and/or extrinsic activators. Preferably, the extrinsic activator of coagulation is the Tissue Factor (TF). This Tissue Factor preferably is selected from lipidated TF or recombinant TF. The intrinsic activator of coagulation preferably is selected from celite, ellagic acid, sulfatit, kaolin, silica, RNA or mixtures thereof. The coagulation activator can further be selected from one or more coagulation factors and/or activated coagulation factors, preferably FXa, FVa, activated protein C, or FVIIa.

In a further embodiment, prior to the determination performed in steps c) and d) one or more of the following ingredients are added to the blood sample: at least one activator of coagulation (see above); a calcium salt, preferably CaCl₂; one or more coagulation components or factors.
The calcium salt is added to recalcify the blood sample if citrated whole blood has been used as blood sample of said patient. CaCl₂ is preferably used in a proper amount to ensure recalcification of the sample. It turned out that the amount of from 3 - 30 µmol/ml is optimal to achieve this requirement. In order to determine the required amount of CaCl₂ to be contained in the diagnostic composition even more precisely, the exact volume of the test liquid to be collected from the patient has to be known as well as the amount of the decalcifying reagent (e.g., citrate) employed.

In a further embodiment, the contribution of the agent which is able to polymerize to the overall viscoelastic behaviour of the blood clot determined in steps c) and d) is subtracted from the results by a software algorithm to ensure comparability of the obtained results with presently available diagnostic results and therapeutic guidelines.

## Claims

1. A diagnostic method for the determination of a coagulopathy in a patient, comprising the steps of:
a) obtaining a blood sample from a patient;
b) adding a coagulation component inhibitor to the sample in a suitable amount for inhibiting said coagulation component;
c) performing a viscoelastometric measurement in the blood sample under suitable conditions in a suitable apparatus;
d) comparing the results obtained in step d) with those from other donors;
wherein the results are indicative for the presence of a coagulopathy and enable the assessment of amount of coagulation component to be administered to the patient to balance haemostasis.

2. The method of claim 1, wherein the viscoelastometric measurement performed in step c) comprises the determination of at least one of the following coagulation characteristics: clotting time, clot formation time, firmness of the clot over time, maximum clot firmness or fibrinolysis extent.

3. The method of claim 1, wherein the coagulation component inhibitor is a platelet inhibitor and is selected from the group of cyto-skeletton inhibitors and/or GPIIb/IIIa antagonists, and preferably is cytochalasin D, abciximab, or a mixture thereof.

4. The method of claim 1, wherein prior to step c) a further agent able to polymerize and thereby to increase the blood clot firmness is added in a predefined amount to the sample.

5. The method of claim 4, wherein the agent which is able to polymerize and thereby to increase the blot clot firmness is fibrinogen.

6. The method of one or more of claims 1-5, wherein, subsequently, a further diagnostic method is performed,
wherein steps c) and d) are applied to untreated blood samples, wherein differing results are indicative for the presence of a coagulopathy.

7. The method of one or more of claims 1-6, wherein from the results obtained in step d) and optionally based on further parameters, the amount of blood components to be substituted to the patient is calculated.

8. The method of claim 7, wherein the further parameters comprise the overall blood volume and the body weight of the patient.

9. The method of claim 7 or 8, wherein the blood components comprise blood platelets and/or fibrinogen.

10. The method of one or more of the preceding claims, wherein the determination in step c) and d) is performed by an apparatus suitable for performing a viscoelastometric analysis.

11. The method of claim 10, wherein the apparatus is a thromboelastometer or a thrombelastograph.

12. The method of one or more of the preceding claims wherein the blood sample is obtained from a mammal, preferably from a human patient.

13. The method of claim 12, wherein the blood sample is whole blood or blood plasma.

14. The method of claim 4, wherein an activator of coagulation is added to the blood sample.

15. The method of one or more of the preceding claims, wherein prior to the determination performed in steps c) and d), one or more of the following ingredients are added to the blood sample:
- at least one activator of coagulation;
- a calcium salt, preferably CaCl₂;
- one or more coagulation components or factors.

16. The method of claim 15, wherein the activator of coagulation is an intrinsic and/or extrinsic activator.

17. The method of claim 15 or 16, wherein the extrinsic activator of coagulation is the Tissue Factor (TF).

18. The method of claim 17, wherein the Tissue Factor is selected from lipidated TF or rTF.

19. The method of claims 15 or 16, wherein the intrinsic activator of coagulation is selected from the group consisting of celite, ellagic acid, sulfatit, kaolin, silica, RNA, or mixtures thereof.

20. The method of one or more of claims 14-19, wherein the activator of coagulation is selected from one or more coagulation factors or activated coagulation factors, preferably FXa or FVa or activated protein C or FVIIa.

21. The method of one or more of claims 14-20, wherein CaCl₂ is present in an amount of about 1-100 µmol/ml of the blood sample.

22. The method of one or more of the preceding claims, wherein the contribution of the agent, which is able to polymerize, to the viscoelastic parameters determined in steps c) and d), is subtracted from the results by a suitable algorithm.
